# EUROPEAN PATENT SPECIFICATION

(11) **EP 4 109 221 B1**
(45) Date of publication and mention of the grant of the patent: **23.09.2026**
(21) Application number: 22158647.2
(22) Date of filing: 24.02.2022
(51) Int. Cl.: G06F 3/01, A61B 3/10, A61F 9/08, A61B 3/113, G02B 27/01, G02C 11/00

(54) **HEAD-MOUNTED ELECTRONIC VISION AID DEVICE AND VISUAL DISTORTION CORRECTION METHOD THEREOF**
AM KOPF MONTIERTE ELEKTRONISCHE SEHHILFEVORRICHTUNG UND VERFAHREN ZUR KORREKTUR DER VISUELLEN VERZERRUNG
DISPOSITIF D'AIDE À LA VISION ÉLECTRONIQUE MONTÉ SUR LA TÊTE ET SON PROCÉDÉ DE CORRECTION DE LA DISTORSION VISUELLE

(30) Priority: 25.06.2021 CN 202110712434
(43) Date of publication of application: 28.12.2022
(73) Proprietor: Artheia Technologies (Suzhou) Co., Ltd., Suzhou, Jiangsu (CN)
(72) Inventor: JIANG, Haotian, Suzhou (CN)
(74) Representative: Kuttenkeuler, David

(56) References cited:
- US-A1- 2014 210 970
- US-A1- 2019 385 342

## Description

### Technical Field

The present invention relates to the field of intelligent medical equipment, in particular to a head-mounted electronic vision aid device and a visual distortion correction method thereof.

### Background art

Macular degeneration is a common medical disease, which is especially common in the elderly. People with macular degeneration usually have damaged or dysfunctional retina (especially the macula of the retina). The macula usually has the highest resolution of eyes. Usually, the damaged or dysfunctional macula may not respond normally to the light in front of a person. As a result, a macular degeneration patient may have blurred vision or no vision at the center of the visual field or a certain area around it. The macular degeneration may limit the abilities of face identification, driving, reading and other aspects of the person. The deterioration of the macula may extend outward from the center of the retina, however, it is unlikely to affect the peripheral area of the retina. Usually, in the early stages of the macular degeneration, the patients will experience decreased vision and visual distortion.

With the technological development of AR/VR wearable devices, more and more head-mounted devices (HMD) are designed to capture images in surrounding scenes of users, display the captured scenes in real time through AR display systems, and can amplify or modify the displayed scenes to assist in enhancing the vision of the macular degeneration patients. In the prior art, the HMD only addresses the problem of black shadow occlusion at the centers of the visual fields of the macular degeneration patients in the middle and advanced stages, the images of the black shadow occlusion areas are presented in the visual fields of the patients through image capturing and amplifying units to assist in enhancing the vision of the patients, but there is no HMD for assisting the patients with visual distortion symptoms in the early stages of macular degeneration to enhance their vision. Therefore, there is an urgent need for a head-mounted electronic vision aid device with a visual distortion correction function to solve the deficiencies in the prior art.

Document US 2019/385342 A1 relates to a wearable mixed reality system comprising a camera input system, and image projection system capable of being worn by a user, and a processor in communication with the camera input system and the image projection system. The processor may be capable of receiving a real-world image from the camera input system and simultaneously displaying at least a portion of the real-world image and an augmented image on the image projection system such that a user views the portion of the real-world image and the augmented image simultaneously.

### Summary of the Invention

In order to solve the technical problem that a head-mounted electronic vision aid device in the prior art has no visual distortion correction function, the present invention aims to provide a head-mounted electronic vision aid device, which can intuitively achieve visual distortion correction with convenient operations for different vision demands of users, and a visual distortion correction method.

The invention is defined by the appended independent claims. Further embodiments of the invention are defined by the dependent claims.

In order to achieve one of the above-mentioned purposes of the present invention, one embodiment of the present invention provides a head-mounted electronic vision aid device, including a user input unit, a storage unit, a processing unit and a display unit, characterized in that: the display unit is configured to movably display a grid/grid group, the user input unit is configured to select at least one to-be-corrected part in the grid/grid group according to the real-time image feedback information viewed by a user, when the to-be-corrected part is selected, the user input unit sends a correction control signal for performing image correction on the to-be-corrected part in a coordinate system where the grid/grid group is currently located to the processing unit, the processing unit performs calculation processing on the correction control signal to obtain variations of the to-be-corrected part before and after correction in the coordinate system, the storage unit stores plurality of combinations of the variations of the to-be-corrected part, and the display unit can display an image after visual distortion correction according to the plurality of combinations of the variations stored in the storage unit.

As a further improvement of one embodiment of the present invention, the processing unit is configured to control the grid/grid group of the coordinate system to automatically move a set stepping distance within the maximum interval range of any adjacent grids.

As a further improvement of one embodiment of the present invention, the user input unit can also send a movement control signal for moving the grid/grid group in the coordinate system to the processing unit, and the processing unit is configured to control the grid/grid group displayed by the display unit to move on demand within the maximum interval range of any adjacent grids according to the movement control signal.

As a further improvement of one embodiment of the present invention, the head-mounted electronic vision aid device further includes an eye-tracking unit, when the eye-tracking unit detects a change in the gazing target of the user, the coordinate system where the grid/grid group is displaced synchronously following the gazing target, and the display unit applies the plurality of combinations of the variations stored in the storage unit on the coordinate system after the synchronous displacement, and displays the image after visual distortion correction.

As a further improvement of one embodiment of the present invention, the user input unit can be a mouse, a remote controller, a button, a gesture recognition device, or a voice recognition device.

As a further improvement of one embodiment of the present invention, the correction control signal includes a signal for causing one or more sides of the to-be-corrected part to bend, elongate or tilt; or a signal for adjusting the brightness, contrast and tone of the to-be-corrected part; or a signal for zooming in or zooming out the to-be-corrected part.

As a further improvement of one embodiment of the present invention, the distance between adjacent grids of the grid/grid group can be adjusted on demand.

The present invention further provides a visual distortion correction method of a head-mounted electronic vision aid device. The device includes a user input unit, a storage unit, a processing unit and a display unit, and the method includes the following steps:
A: the display unit movably displays a grid/grid group;
B: a user selects at least one to-be-corrected part in the grid/grid group through the user input unit according to the real-time image feedback information viewed by the display unit;
C: the user input unit sends a correction control signal for performing image correction on the to-be-corrected part in a coordinate system where the grid/grid group is currently located to the processing unit;
D: the processing unit performs calculation processing on the correction control signal to obtain variations of the to-be-corrected part before and after correction in the coordinate system;
E: the storage unit stores plurality of combinations of the variations of the to-be-corrected part; and
F: the display unit displays an image after visual distortion correction according to the plurality of combinations of the variations stored in the storage unit.

As a further improvement of one embodiment of the present invention, the method further includes a step C1 between the steps C and D: the processing unit controls the grid/grid group of the coordinate system to automatically move a set stepping distance within the maximum interval range of any adjacent grids for further automatic correction of all to-be-corrected parts of the coordinate system.

As a further improvement of one embodiment of the present invention, the method further includes a step C1 between the steps C and D: the user input unit sends a movement control signal for moving the grid/grid group in the coordinate system to the processing unit, and the processing unit controls the grid/grid group displayed by the display unit to move on demand within the maximum interval range of any adjacent grids according to the movement control signal, so as to further correct all to-be-corrected parts of the coordinate system on demand.

As a further improvement of one embodiment of the present invention, the device further includes an eye-tracking unit, when the eye-tracking unit detects a change in the gazing target of the user, the coordinate system where the grid/grid group is displaced synchronously following the gazing target, and the display unit applies the plurality of combinations of the variations stored in the storage unit on the coordinate system after the synchronous displacement, and displays the image after visual distortion correction.

As a further improvement of one embodiment of the present invention, in the step B, the user input unit can be a mouse, a remote controller, a button, a gesture recognition device, or a voice recognition device, and the outer edge of the to-be-corrected part is selected by the user input unit to select the to-be-corrected part.

As a further improvement of one embodiment of the present invention, the method further includes a confirmation step for the user to confirm the correction control signal between the step C and the step C1.

As a further improvement of one embodiment of the present invention, the correction control signal includes a signal for causing one or more sides of the to-be-corrected part to bend, elongate or tilt; or a signal for adjusting the brightness, contrast and tone of the to-be-corrected part; or a signal for zooming in or zooming out the to-be-corrected part.

As a further improvement of one embodiment of the present invention, the distance between adjacent grids of the grid/grid group can be adjusted on demand.

As a further improvement of one embodiment of the present invention, the storage unit in the step E can store the plurality of combinations of the variations of the to-be-corrected parts of different users or of the same user at different time points, and the user can repeat the steps A to F on the basis of calling the plurality of combinations.

Compared with the prior art, the prevent invention has the beneficial effects that: by introducing the movable grid/grid group into the display unit of the head-mounted electronic vision aid device to establish a basic correction coordinate system, the user can perform correction and calibration of visual distortion after selecting the to-be-corrected part according to his own vision status, the processing unit performs calculation processing on the correction control signal to obtain the variations of the to-be-corrected part before and after correction in the basic correction coordinate system, all to-be-corrected parts within the visual field can be corrected just by moving within the maximum interval range of any adjacent grids, the storage unit stores the plurality of combinations of the variations of all to-be-corrected parts, the display unit can display the image after visual distortion correction according to the plurality of combinations of the variations stored in the storage unit and verify the corrected image in real time, the user can quickly and intuitively realize the image correction of visual distortion with convenient operations, and the demands of low-vision users with different symptom progresses are met.

### Brief Description of the Drawings

Fig. 1 is a schematic diagram of a hardware module of one embodiment of a head-mounted electronic vision aid device 100;
Fig. 2 is a schematic diagram of software flow of one embodiment of the head-mounted electronic vision aid device 100;
Fig. 3 is a schematic structural diagram of one embodiment of the head-mounted electronic vision aid device 100;
Fig. 4 is a schematic structural diagram of a selected frame of determining a target object of interest to a user in the head-mounted electronic vision aid device 100;
Fig. 5 is a schematic diagram of a correction operation of the head-mounted electronic vision aid device 100 when a to-be-corrected part is discovered for the first time; and
Fig. 6 is a schematic diagram of the correction operation of the head-mounted electronic vision aid device 100 when the to-be-corrected part is discovered for the second time.

In which:
100, head-mounted electronic vision aid device; 101, user input unit; 102, storage unit; 103, processing unit; 104, display unit; 105, eye-tracking unit; 106, to-be-corrected part; 107, grid/grid group; 108, central point.

### Detailed Description of the Embodiments

The embodiments of the present invention are described in detail below. Examples of the embodiments are shown in the drawings, in which the same or similar reference signs indicate the same or similar elements or elements with the same or similar functions. The embodiments described below with reference to the drawings are exemplary, and are intended to explain the present invention, but cannot be construed as limitations to the present invention.

The technical solutions of the present invention will be further described below in conjunction with the drawings and specific embodiments.

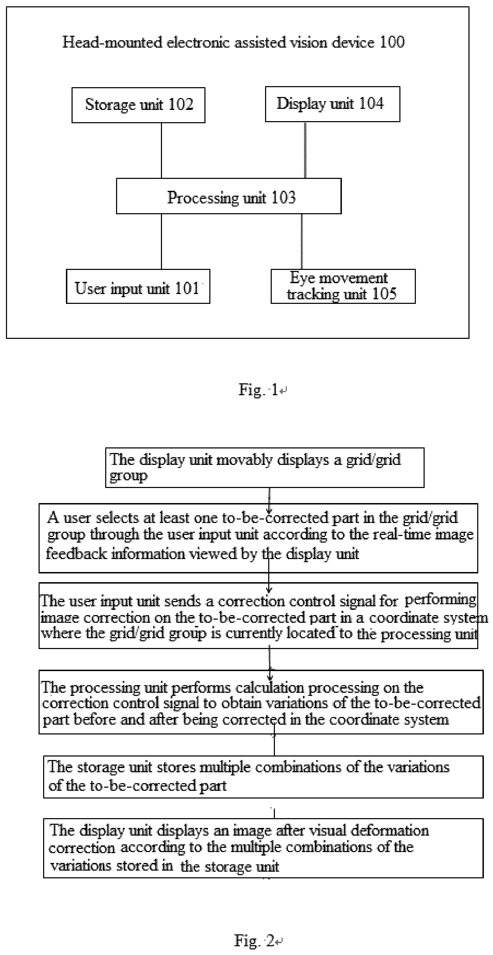

Referring to Fig. 1, embodiments of the present invention are provided to achieve one of the above-mentioned objectives of the present invention. One embodiment of the present invention provides a head-mounted electronic vision aid device 100, including a user input unit 101, a storage unit 102, a processing unit 103 and a display unit 104. The processing unit 103 can be operatively connected to a database through a link connected to an input/output (I/O) circuit. The storage unit 102 can include a plurality of RAMs and a plurality of program memories, and the RAMs and the program memories can be, for example, semiconductor memories, magnetically readable memories, and/or optically readable memories.

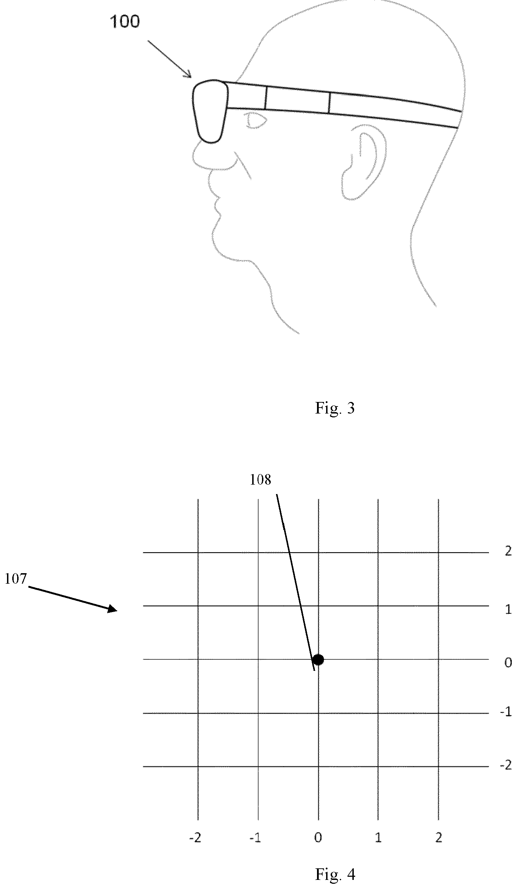

Referring to Fig. 3, the head-mounted electronic vision aid device 100 can be AR augmented reality type vision-aid glasses that integrate optical and electrical hardware and software, and an amplified and corrected image can be displayed on the display unit of the head-mounted AR glasses and provided for a user. In other alternative embodiments, the head-mounted electronic vision aid device 100 can also form communication coupling with one or more external image processors through wired or wireless communication, and the external image processor can include a computer, such as a laptop computer, a tablet computer, a mobile communication terminal device and other computer processing devices, and the external image processor can include one or more processors and one or more memories. In the embodiment including the external image processor, the image undergoing correction processing is sent to the display unit of the head-mounted electronic vision aid device 100 for the viewing of a subject.

Referring to Fig. 4, the display unit 104 is configured to movably display a grid/grid group 107, the grid spacing of the grid/grid group 107 of the present invention can be adjusted on demand, and is preferably a spacing that is easy for the eyes of low-vision users to distinguish whether the lines are curved, instead of a spacing that is too small. For example, the grid/grid group 107 can have a coordinate identifier as shown in the figure, and in this coordinate system, the coordinates of a central point are (0, 0).

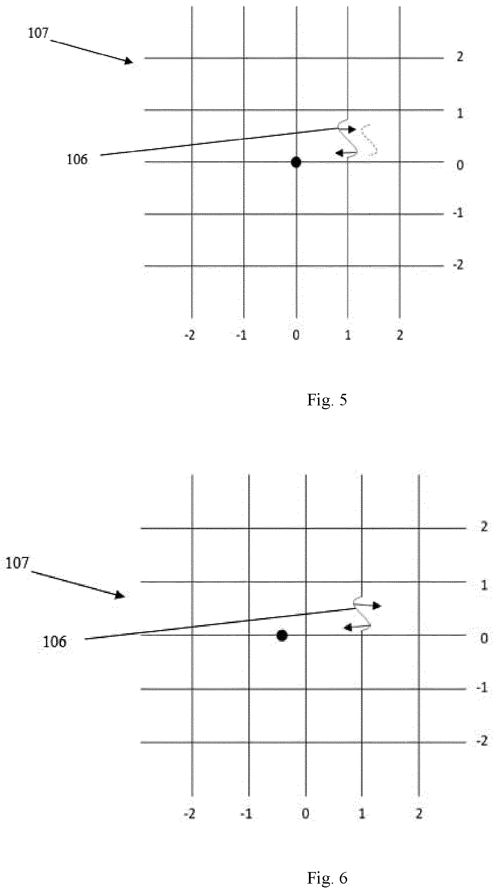

Referring to Fig. 5, when a correction operation is started, the user should look at the central point 108 of the grid/grid group 107 with both eyes as required to determine a basic coordinate system of the correction operation. The user input unit 101 is configured to select at least one to-be-corrected part 106 in the grid/grid group 107 according to the real-time image feedback information viewed by the user. If it is assumed that when the user starts the first correction operation, the user discovers that a vertical line of the grid/grid group 107 is obviously deformed (at this time, a second obviously deformed to-be-corrected part displayed by the dashed line is not located on the grid/grid group, so that the user cannot see it). At this time, the user can operate the user input unit 101, such as a mouse, a remote controller, a button, a gesture recognition device, or a voice recognition device to select the obviously deformed part and restore the obviously deformed part into a straight line within the visual field of the user, and the selection operation can be that the outer edge of the to-be-corrected part is highlighted or is changed into a high-contrast color. During the correction operation, the upper part of the to-be-corrected part 106 bent in an S shape shown in Fig. 5 is dragged rightward, the lower part of the to-be-corrected part 106 bent in the S shape is dragged leftward, and then fine tuning is performed to change the to-be-corrected part 106 into a straight line.

When the user selects the to-be-corrected part 106 and performs the correction operation, the user input unit 101 sends a correction control signal for performing image correction on the to-be-corrected part 106 in a coordinate system where the grid/grid group 107 is currently located to the processing unit 103, and the correction control signal includes, but is not limited to, a signal for causing one or more sides of the to-be-corrected part 106 to bend, elongate or tilt; or a signal for adjusting the brightness, contrast and tone of the to-be-corrected part 106; or a signal for zooming in or zooming out the to-be-corrected part 106.

The processing unit 103 performs calculation processing on the correction control signal to obtain variations of the to-be-corrected part 106 before and after correction in the coordinate system, for example, the variations can be a plurality of coordinate variable arrays, the storage unit 102 stores plurality of combinations of the variations of the to-be-corrected part 106, and the display unit 104 can display an image after visual distortion correction according to the plurality of combinations of the variations stored in the storage unit 102, that is, the image after the visual distortion correction according to the eye parameters of the user. Compared with the prior art, by introducing the movable grid/grid group into the display unit of the head-mounted electronic vision aid device to establish the basic correction coordinate system, the user can perform correction and calibration of visual distortion after selecting the to-be-corrected part according to his own vision status, the processing unit performs the calculation processing on the correction control signal to obtain the variations of the to-be-corrected part before and after correction in the basic correction coordinate system, all to-be-corrected parts within the visual field can be corrected just by moving within the maximum interval range of any adjacent grids, the storage unit stores the plurality of combinations of the variations of all to-be-corrected parts, the display unit can display the image after visual distortion correction according to the plurality of combinations of the variations stored in the storage unit and verify the corrected image in real time, the user can quickly and intuitively realize the image correction of visual distortion with convenient operations, and the demands of low-vision users with different symptom progresses are met.

In a preferred embodiment, the processing unit 103 is configured to control the grid/grid group 107 of the coordinate system to automatically move a set stepping distance within the maximum interval range of any adjacent grids. That is, the user can select an automatic stepping correction mode. In the automatic stepping correction mode, the grid/grid group 107 is automatically moved leftward/rightward or up/down by the set distance in the initial basic coordinate system, when the user discovers again that there are deformed vertical or horizontal lines in the moved grid/grid group 107 (at this time, the to-be-corrected part shown by the dashed line in Fig. 5 is located on the grid/grid group, and thus is visible to the user), referring to Fig. 6, the user input unit 101 is operated again to correct the newly discovered to-be-corrected part 106 again to make it straight. In this way, all to-be-corrected parts within the visual field can be corrected just by automatically moving within the maximum interval range of any adjacent grids for several times. The low-vision users can be intuitively assisted in correcting the visual distortion within the visual field, the correction effect is verified at the same time, and the operation is convenient.

In another preferred embodiment, the user can also select a manual stepping correction mode, that is, the user manually controls the user input unit 101 to send a movement control signal for moving the grid/grid group 107 in the coordinate system to the processing unit 103 according to his own demand, and the processing unit 103 is configured to control the grid/grid group 107 displayed by the display unit 104 according to the movement control signal to move on demand within the maximum interval range of any adjacent grids. After the grid/grid group 107 is automatically moved within the maximum interval range of any adjacent grids, when the user discovers deformed vertical or horizontal lines in the moved grid/grid group 107, referring to Fig. 6, the user input unit 101 is operated again to correct the newly discovered to-be-corrected part 106 again to make it straight. In this way, all to-be-corrected parts within the visual field can be corrected just by moving within the maximum interval range of any adjacent grids for several times. The low-vision users can be intuitively assisted in correcting the visual distortion within the visual field, the correction effect is verified at the same time, the operation is convenient, and the user experience is better.

The head-mounted electronic vision aid device 100 of the present invention further includes an eye-tracking unit 105, when the eye-tracking unit 105 detects a change in the gazing target of the user, the coordinate system where the grid/grid group 107 is displaced synchronously following the gazing target, and the display unit 104 applies the plurality of combinations of the variations stored in the storage unit 102 on the coordinate system after the synchronous displacement, and displays the image after visual distortion correction. In other words, after the user has corrected all to-be-corrected parts, at this time, the user no longer needs to stare at the central point 108 of the displayed grid/grid group 107, the coordinate system of the grid/grid group 107 can follow the gazing target detected by the eye-tracking unit 105 to change synchronously, the user can turn his eyes, so that the plurality of combinations of the variations of the previously corrected to-be-corrected part 106 are applied in real time to a new coordinate system with the grid/grid group 107, and the real-time verification of the correction effect is achieved during the correction. Furthermore, in the case of fine tuning of the gazing target during the use of the user, the eye-tracking unit 105 causes the coordinate system to move synchronously with the gazing target, which ensures the consistency of the correction operation of the user and the accuracy of the adjustment.

The user described in the present invention includes low-vision users, such as macular degeneration patients in early stages, age-related macular degeneration (AMD), macular hole and other FOV field-related blindness or vision defects, such as central macular scar, histoplasmosis, advanced stage glaucoma, macular degeneration, central serous retinopathy, myopic macular degeneration, diabetic macular edema, bladder ID macular edema, macular hole, macular atrophy, central macular scar, histoplasmosis, macular hole, anterior ischemic optic neuropathy and retinitis pigmentosa, and so on. The to-be-corrected part 106 in the image in the visual field usually has different degrees of distortion, including, but not limited to, swirling distortion, barrel distortion, or pillow distortion.

Referring to 2, the present invention further visual distortion correction method of a head-mounted electronic vision aid device 100. The head-mounted electronic vision aid device 100 includes a user input unit 101, a storage unit 102, a processing unit 103 and a display unit 104, and the method includes the following steps:
A: the display unit 104 movably displays a grid/grid group 107;
B: a user selects at least one to-be-corrected part 106 in the grid/grid group 107 through the user input unit 101 according to the real-time image feedback information viewed by the display unit 104;
C: the user input unit 101 sends a correction control signal for performing image correction on the to-be-corrected part 106 in a coordinate system where the grid/grid group 107 is currently located to the processing unit 103;
D: the processing unit 103 performs calculation processing on the correction control signal to obtain variations of the to-be-corrected part 106 before and after correction in the coordinate system;
E: the storage unit 102 stores plurality of combinations of the variations of the to-be-corrected part 106; and
F: the display unit 104 can display an image after visual distortion correction according to the plurality of combinations of the variations stored in the storage unit 102.

The grid spacing of the grid/grid group 107 of the present invention can be adjusted on demand, and is preferably a spacing that is easy for the eyes of low-vision users to distinguish whether the lines are curved, instead of a spacing that is too small, so as to avoid the situation that the low-vision users are difficult to distinguish and operation. When a correction operation is started, the user should look at the central point 108 of the grid/grid group 107 with both eyes as required to determine a basic coordinate system of the correction operation. Referring to Fig. 5, if it is assumed that when the user starts the first correction operation, the user discovers that a vertical line of the grid/grid group 107 is obviously deformed, at this time, the user can operate the user input unit 101, such as a mouse, a remote controller, a button, a gesture recognition device, or a voice recognition device to select the obviously deformed part and restore the obviously deformed part into a straight line within the visual field of the user. For example, the upper part of the to-be-corrected part 106 bent in an S shape shown in FIG. 5 is dragged rightward, the lower part of the to-be-corrected part 106 bent in the S shape is dragged leftward, and then fine tuning is performed to change the to-be-corrected part 106 into a straight line.

When the user selects the to-be-corrected part 106 and performs the correction operation, the user input unit 101 sends a correction control signal for performing image correction on the to-be-corrected part 106 in a coordinate system where the grid/grid group 107 is currently located to the processing unit 103, and the correction control signal includes, but is not limited to, a signal for causing one or more sides of the to-be-corrected part 106 to bend, elongate or tilt; or a signal for adjusting the brightness, contrast and tone of the to-be-corrected part 106; or a signal for zooming in or zooming out the to-be-corrected part 106.

The processing unit 103 performs calculation processing on the correction control signal to obtain variations of the to-be-corrected part 106 before and after correction in the coordinate system, for example, the variations can be a plurality of coordinate variable arrays, the storage unit 102 stores plurality of combinations of the variations of the to-be-corrected part 106, and the display unit 104 can display an image after visual distortion correction according to the plurality of combinations of the variations stored in the storage unit 102, that is, the image after the visual distortion correction according to the eye parameters of the user.

In a preferred embodiment, the processing unit 103 controls the grid/grid group 107 of the coordinate system to automatically move a set stepping distance within the maximum interval range of any adjacent grids, for example, three times of stepping fine tuning are uniformly arranged between two adjacent grids. That is, the user can select an automatic stepping correction mode. In the automatic stepping correction mode, the grid/grid group 107 is automatically moved leftward/rightward or up/down by the set distance in the initial basic coordinate system, when the user discovers again that there are deformed vertical or horizontal lines in the moved grid/grid group 107, referring to Fig. 6, the user input unit 101 is operated again to correct the newly discovered to-be-corrected part 106 again to make it straight. In this way, all to-be-corrected parts within the visual field can be corrected just by automatically moving within the maximum interval range of any adjacent grids for several times. The low-vision users can be intuitively assisted in correcting the visual distortion within the visual field, the correction effect is verified at the same time, and the operation is convenient.

In another preferred embodiment, the user can also select a manual stepping correction mode, that is, the user manually fine tunes and controls the user input unit 101 to send a movement control signal for moving the grid/grid group 107 in the coordinate system to the processing unit 103 according to his own demand, and the processing unit 103 controls the grid/grid group 107 displayed by the display unit 104 according to the movement control signal to move on demand within the maximum interval range of any adjacent grids. After the grid/grid group 107 is automatically moved within the maximum interval range of any adjacent grids, when the user discovers deformed vertical or horizontal lines in the moved grid/grid group 107, referring to Fig. 6, the user input unit 101 is operated again to correct the newly discovered to-be-corrected part 106 again to make it straight. In this way, all to-be-corrected parts within the visual field can be corrected just by moving within the maximum interval range of any adjacent grids for several times. The low-vision users can be intuitively assisted in correcting the visual distortion within the visual field, the correction effect is verified at the same time, and the operation is convenient.

The head-mounted electronic vision aid device 100 of the present invention further includes an eye-tracking unit 105, when the eye-tracking unit detects a change in the gazing target of the user, the coordinate system where the grid/grid group 107 is displaced synchronously following the gazing target, and the display unit 104 applies the plurality of combinations of the variations stored in the storage unit 102 on the coordinate system after the synchronous displacement, and displays the image after visual distortion correction. In other words, after the user has corrected all to-be-corrected parts, at this time, the user no longer needs to stare at the central point 108 of the displayed grid/grid group 107, the coordinate system of the grid/grid group 107 can follow the gazing target detected by the eye-tracking unit 105 to change synchronously, the user can turn his eyes, so that the plurality of combinations of the variations of the previously corrected to-be-corrected part 106 are applied in real time to a new coordinate system with the grid/grid group 107, and the real-time verification of the correction effect is achieved during the correction. Furthermore, in the case of fine tuning of the gazing target during the use of the user, the eye-tracking unit 105 causes the coordinate system to move synchronously with the gazing target, which ensures the consistency of the correction operation of the user and the accuracy of the adjustment.

In a preferred embodiment, the method further includes a confirmation step for the user to confirm the correction control signal between the step C and the step C1, and the next step is performed until the user is satisfied with the distortion correction effect.

The storage unit 102 in the step E can store plurality of combinations of the variations of the to-be-corrected parts 106 of different users or of the same user at different time points, and the user can repeat the steps A to F on the basis of calling the plurality of combinations. In a preferred embodiment, the user can also select and call the data of the plurality of combinations of the variations stored in the previous correction through menu options, and make adjustment again on this basis. For the low-vision users, the visual field change or the development process of eye diseases can also be judged based on the data.

## Claims

1. A head-mounted electronic vision aid device (100), comprising a user input unit (101), a storage unit (102), a processing unit (103), and a display unit (104), wherein:
the display unit (104) is configured to movably display a grid/grid group (107), the user input unit (101) is configured to select one or more to-be-corrected parts (106) in the grid/grid group (107) according to real-time image feedback information viewed by a user, **characterized in**
for each of the one or more to-be-corrected parts (106) selected, the user input unit (101) sends a correction control signal for performing image correction on the to-be-corrected part (106) in a coordinate system where the grid/grid group (107) is currently located to the processing unit (103), the correction control signal comprises a signal for causing one or more sides of the to-be-corrected part (106) to bend, elongate or tilt to correct the to-be-corrected part (106) to be a straight line in the coordinate system in a visual field of the user, and the processing unit (103) performs calculation processing on the correction control signal to obtain variations of the to-be-corrected part (106) before and after correction in the coordinate system,
the storage unit (102) stores the one or more combinations of the variations obtained for the one or more to-be-corrected parts, and
the display unit (104) displays an image after visual distortion correction according to the one or more combinations of the variations stored in the storage unit (102).

2. The head-mounted electronic vision aid device according to claim 1, **characterized in that**: the processing unit (103) is configured to control the grid/grid group (107) of the coordinate system to automatically move a set stepping distance within a maximum interval range of any adjacent grid lines.

3. The head-mounted electronic vision aid device according to claim 1, **characterized in that**: the user input unit (101) sends a movement control signal for moving the grid/grid group (107) in the coordinate system to the processing unit (103), and the processing unit (103) is configured to control the grid/grid group (107) displayed by the display unit (104) to move on demand within a maximum interval range of any adjacent grid lines according to the movement control signal.

4. The head-mounted electronic vision aid device according to claim 1, further comprising: an eye-tracking unit (105), when the eye-tracking unit detects a change in the gazing target of the user, the coordinate system where the grid/grid group (107) is displaced synchronously following the gazing target, and the display unit (104) applies the one or more combinations of the variations stored in the storage unit (102) on the coordinate system after the synchronous displacement, and displays the image after visual distortion correction.

5. The head-mounted electronic vision aid device according to claim 1, **characterized in that**: the user input unit (101) is a mouse, a remote controller, a button, a gesture recognition device, or a voice recognition device, and the variations are a plurality of coordinate variable arrays.

6. The head-mounted electronic vision aid device according to claim 1, **characterized in that**: the distance between adjacent grid lines of the grid/grid group (107) is adjusted on demand.

7. A visual distortion correction method of a head-mounted electronic vision aid device (100), the device comprising a user input unit (101), a storage unit (102), a processing unit (103), and a display unit (104), wherein the method comprising the following steps:
A: the display unit (104) movably displays a grid/grid group (107);
B: a user selects one or more to-be-corrected parts (106) in the grid/grid group (107) through the user input unit (101) according to the real-time image feedback information viewed by the user on the display unit (104); **characterized in that** the method comprises:
C: for each of the one or more to-be-corrected parts (106), the user input unit (101) sends a correction control signal for performing image correction on the to-be-corrected part (106) in a coordinate system where the grid/grid group (107) is currently located to the processing unit (103), the correction control signal comprises a signal for causing one or more sides of the to-be-corrected part (106) to bend, elongate or tilt to correct the to-be-corrected part (106) to be a straight line in the coordinate system in a visual field of the user;
D: for each of the one or more to-be-corrected parts (106), the processing unit (103) performs calculation processing on the correction control signal to obtain variations of the to-be-corrected part (106) before and after correction in the coordinate system;
E: the storage unit (102) stores the one or more combinations of the variations obtained for the one or more to-be-corrected parts (106); and
F: the display unit (104) displays an image after visual distortion correction according to the one or more combinations of the variations stored in the storage unit (102).

8. The visual distortion correction method of the head-mounted electronic vision aid device according to claim 7, further comprising:
a step C1 between the steps C and D: the processing unit (103) controls the grid/grid group (107) of the coordinate system to automatically move a set stepping distance within the maximum interval range of any adjacent grid lines for further automatic correction of all to-be-corrected parts (106) of the coordinate system.

9. The visual distortion correction method of the head-mounted electronic vision aid device according to claim 7, further comprising:
a step C1 between the steps C and D: the user input unit (101) sends a movement control signal for moving the grid/grid group (107) in the coordinate system to the processing unit (103), and the processing unit (103) controls the grid/grid group (107) displayed by the display unit (104) to move on demand within a maximum interval range of any adjacent grid lines according to the movement control signal, so as to further correct all to-be-corrected parts (106) of the coordinate system on demand.

10. The visual distortion correction method of the head-mounted electronic vision aid device according to claim 7, **characterized in that**: the device further comprises an eye-tracking unit (105), when the eye-tracking unit detects a change in the gazing target of the user, the coordinate system where the grid/grid group (107) is displaced synchronously following the gazing target, and the display unit (104) applies the one or more combinations of the variations stored in the storage unit (102) on the coordinate system after the synchronous displacement, and displays the image after visual distortion correction.

11. The visual distortion correction method of the head-mounted electronic vision aid device according to claim 7, **characterized in that**: in the step B, the user input unit (101) is a mouse, a remote controller, a button, a gesture recognition device, or a voice recognition device, and the outer edge of the to-be-corrected part (106) is selected by the user input unit (101) to select the to-be-corrected part (106).

12. The visual distortion correction method of the head-mounted electronic vision aid device according to claim 8 or 9, further comprising:
a confirmation step for the user to confirm the correction control signal between the step C and the step C1.

13. The visual distortion correction method of the head-mounted electronic vision aid device according to claim 7, **characterized in that**: the distance between adjacent grid lines of the grid/grid group (107) is adjusted on demand.

14. The visual distortion correction method of the head-mounted electronic vision aid device according to claim 7, **characterized in that**: the storage unit (102) in the step E stores the combinations of the variations of the one or more to-be-corrected parts (106) of different users or of the same user at different time points, and repeating the steps A to F on the basis of calling the one or more combinations.

## Patentansprüche

1. Am Kopf befestigte elektronische Sehhilfevorrichtung (100), umfassend eine Benutzereingabeeinheit (101), eine Speichereinheit (102), eine Verarbeitungseinheit (103) und eine Anzeigeeinheit (104), wobei:
die Anzeigeeinheit (104) konfiguriert ist, um ein Raster/eine Rastergruppe (107) beweglich anzuzeigen, und die Benutzereingabeeinheit (101) konfiguriert ist, um gemäß den von einem Benutzer betrachteten Echtzeit-Bildrückmeldungsinformationen einen oder mehrere zu korrigierende Bereiche (106) in dem Raster/der Rastergruppe (107) auszuwählen,
**dadurch gekennzeichnet, dass**
die Benutzereingabeeinheit (101) für jeden der ausgewählten einen oder mehreren zu korrigierenden Bereiche (106) ein Korrektursteuersignal zum Durchführen einer Bildkorrektur an dem zu korrigierenden Bereich (106) in einem Koordinatensystem, in dem sich das Raster/die Rastergruppe (107) aktuell befindet, an die Verarbeitungseinheit (103) sendet, wobei das Korrektursteuersignal ein Signal umfasst, das bewirkt, dass eine oder mehrere Seiten des zu korrigierenden Bereichs (106) gebogen, verlängert oder geneigt werden, um den zu korrigierenden Bereich (106) im Sichtfeld des Benutzers zu einer geraden Linie im Koordinatensystem zu korrigieren, und die Verarbeitungseinheit (103) eine Berechnungsverarbeitung des Korrektursteuersignals durchführt, um Variationen des zu korrigierenden Bereichs (106) vor und nach der Korrektur im Koordinatensystem zu erhalten,
die Speichereinheit (102) die eine oder mehrere Kombinationen der für den einen oder die mehreren zu korrigierenden Bereiche erhaltenen Variationen speichert, und
die Anzeigeeinheit (104) gemäß der einen oder mehreren in der Speichereinheit (102) gespeicherten Kombinationen der Variationen ein Bild nach einer Korrektur visueller Verzerrungen anzeigt.

2. Am Kopf befestigte elektronische Sehhilfevorrichtung nach Anspruch 1, wobei: die Verarbeitungseinheit (103) konfiguriert ist, um das Raster/die Rastergruppe (107) des Koordinatensystems so zu steuern, dass es/sie sich innerhalb eines maximalen Intervallbereichs beliebiger benachbarter Rasterlinien automatisch um einen festgelegten Schrittabstand bewegt.

3. Am Kopf befestigte elektronische Sehhilfevorrichtung nach Anspruch 1, wobei: die Benutzereingabeeinheit (101) ein Bewegungssteuersignal zum Bewegen des Rasters/der Rastergruppe (107) in dem Koordinatensystem an die Verarbeitungseinheit (103) sendet und die Verarbeitungseinheit (103) konfiguriert ist, um das von der Anzeigeeinheit (104) angezeigte Raster/die Rastergruppe (107) gemäß dem Bewegungssteuersignal auf Anforderung innerhalb eines maximalen Intervallbereichs beliebiger benachbarter Rasterlinien zu bewegen.

4. Am Kopf befestigte elektronische Sehhilfevorrichtung nach Anspruch 1, ferner umfassend eine Blickverfolgungseinheit (105), wobei, wenn die Blickverfolgungseinheit eine Änderung des Blickziels des Benutzers detektiert, das Koordinatensystem, in dem sich das Raster/die Rastergruppe (107) befindet, dem Blickziel folgend synchron verschoben wird und die Anzeigeeinheit (104) die eine oder mehrere in der Speichereinheit (102) gespeicherten Kombinationen der Variationen auf das Koordinatensystem nach der synchronen Verschiebung anwendet und das Bild nach der Korrektur visueller Verzerrungen anzeigt.

5. Am Kopf befestigte elektronische Sehhilfevorrichtung nach Anspruch 1, wobei: die Benutzereingabeeinheit (101) eine Maus, eine Fernbedienung, eine Taste, eine Gestenerkennungsvorrichtung oder eine Spracherkennungsvorrichtung ist und die Variationen aus einer Vielzahl von Arrays von Koordinatenvariablen bestehen.

6. Am Kopf befestigte elektronische Sehhilfevorrichtung nach Anspruch 1, wobei: der Abstand zwischen benachbarten Rasterlinien des Rasters/der Rastergruppe (107) auf Anforderung angepasst wird.

7. Verfahren zur Korrektur visueller Verzerrungen einer am Kopf befestigten elektronischen Sehhilfevorrichtung (100), wobei die Vorrichtung eine Benutzereingabeeinheit (101), eine Speichereinheit (102), eine Verarbeitungseinheit (103) und eine Anzeigeeinheit (104) umfasst, wobei das Verfahren die folgenden Schritte umfasst:
A: bewegliches Anzeigen eines Rasters/einer Rastergruppe (107) durch die Anzeigeeinheit (104);
B: Auswählen eines oder mehrerer zu korrigierender Bereiche (106) in dem Raster/der Rastergruppe (107) durch einen Benutzer mittels der Benutzereingabeeinheit (101) gemäß den von dem Benutzer auf der Anzeigeeinheit (104) betrachteten Echtzeit-Bildrückmeldungsinformationen; wobei das Verfahren Folgendes umfasst:
C: für jeden der einen oder mehreren zu korrigierenden Bereiche (106), Senden eines Korrektursteuersignals durch die Benutzereingabeeinheit (101) an die Verarbeitungseinheit (103) zum Durchführen einer Bildkorrektur an dem zu korrigierenden Bereich (106) in einem Koordinatensystem, in dem sich das Raster/die Rastergruppe (107) aktuell befindet, wobei das Korrektursteuersignal ein Signal umfasst, das bewirkt, dass eine oder mehrere Seiten des zu korrigierenden Bereichs (106) gebogen, verlängert oder geneigt werden, um den zu korrigierenden Bereich (106) im Sichtfeld des Benutzers zu einer geraden Linie in dem Koordinatensystem zu korrigieren;
D: für jeden der einen oder mehreren zu korrigierenden Bereiche (106), Durchführen einer Berechnungsverarbeitung des Korrektursteuersignals durch die Verarbeitungseinheit (103), um Variationen des zu korrigierenden Bereichs (106) vor und nach der Korrektur in dem Koordinatensystem zu erhalten;
E: Speichern der einen oder mehreren Kombinationen der für den einen oder die mehreren zu korrigierenden Bereiche (106) erhaltenen Variationen durch die Speichereinheit (102); und
F: Anzeigen eines Bildes nach der Korrektur visueller Verzerrungen durch die Anzeigeeinheit (104) gemäß der einen oder mehreren in der Speichereinheit (102) gespeicherten Kombinationen der Variationen.

8. Verfahren zur Korrektur visueller Verzerrungen der am Kopf befestigten elektronischen Sehhilfevorrichtung nach Anspruch 7, ferner umfassend:
einen Schritt C1 zwischen den Schritten C und D: wobei die Verarbeitungseinheit (103) das Raster/die Rastergruppe (107) des Koordinatensystems so steuert, dass es/sie sich innerhalb des maximalen Intervallbereichs beliebiger benachbarter Rasterlinien automatisch um einen festgelegten Schrittabstand bewegt, um alle zu korrigierenden Bereiche (106) des Koordinatensystems weiter automatisch zu korrigieren.

9. Verfahren zur Korrektur visueller Verzerrungen der am Kopf befestigten elektronischen Sehhilfevorrichtung nach Anspruch 7, ferner umfassend:
einen Schritt C1 zwischen den Schritten C und D: wobei die Benutzereingabeeinheit (101) ein Bewegungssteuersignal zum Bewegen des Rasters/der Rastergruppe (107) in dem Koordinatensystem an die Verarbeitungseinheit (103) sendet und die Verarbeitungseinheit (103) das von der Anzeigeeinheit (104) angezeigte Raster/die Rastergruppe (107) gemäß dem Bewegungssteuersignal auf Anforderung innerhalb eines maximalen Intervallbereichs beliebiger benachbarter Rasterlinien bewegt, um alle zu korrigierenden Bereiche (106) des Koordinatensystems auf Anforderung weiter zu korrigieren.

10. Verfahren zur Korrektur visueller Verzerrungen der am Kopf befestigten elektronischen Sehhilfevorrichtung nach Anspruch 7,
**dadurch gekennzeichnet, dass**: die Vorrichtung ferner eine Blickverfolgungseinheit (105) umfasst, wobei, wenn die Blickverfolgungseinheit eine Änderung des Blickziels des Benutzers detektiert, das Koordinatensystem, in dem sich das Raster/die Rastergruppe (107) befindet, dem Blickziel folgend synchron verschoben wird und die Anzeigeeinheit (104) die eine oder mehrere in der Speichereinheit (102) gespeicherten Kombinationen der Variationen auf das Koordinatensystem nach der synchronen Verschiebung anwendet und das Bild nach der Korrektur visueller Verzerrungen anzeigt.

11. Verfahren zur Korrektur visueller Verzerrungen der am Kopf befestigten elektronischen Sehhilfevorrichtung nach Anspruch 7,
**dadurch gekennzeichnet, dass**: in Schritt B die Benutzereingabeeinheit (101) eine Maus, eine Fernbedienung, eine Taste, eine Gestenerkennungsvorrichtung oder eine Spracherkennungsvorrichtung ist und der Außenrand des zu korrigierenden Bereichs (106) mittels der Benutzereingabeeinheit (101) ausgewählt wird, um den zu korrigierenden Bereich (106) auszuwählen.

12. Verfahren zur Korrektur visueller Verzerrungen der am Kopf befestigten elektronischen Sehhilfevorrichtung nach Anspruch 8 oder 9, ferner umfassend:
einen Bestätigungsschritt zwischen Schritt C und Schritt C1, in dem der Benutzer das Korrektursteuersignal bestätigt.

13. Verfahren zur Korrektur visueller Verzerrungen der am Kopf befestigten elektronischen Sehhilfevorrichtung nach Anspruch 7,
**dadurch gekennzeichnet, dass**: der Abstand zwischen benachbarten Rasterlinien des Rasters/der Rastergruppe (107) auf Anforderung angepasst wird.

14. Verfahren zur Korrektur visueller Verzerrungen der am Kopf befestigten elektronischen Sehhilfevorrichtung nach Anspruch 7,
**dadurch gekennzeichnet, dass**: die Speichereinheit (102) in Schritt E die Kombinationen der Variationen des einen oder der mehreren zu korrigierenden Bereiche (106) verschiedener Benutzer oder desselben Benutzers zu unterschiedlichen Zeitpunkten speichert und die Schritte A bis F auf der Grundlage des Abrufens der einen oder mehreren Kombinationen wiederholt werden.

## Revendications

1. Dispositif d'aide à la vision électronique monté sur tête (100), comprenant une unité d'entrée utilisateur (101), une unité de stockage (102), une unité de traitement (103) et une unité d'affichage (104), dans lequel :
l'unité d'affichage (104) est configurée pour afficher de manière mobile une grille/un groupe de grilles (107), l'unité d'entrée utilisateur (101) est configurée pour sélectionner une ou plusieurs parties à corriger (106) dans la grille/le groupe de grilles (107) selon des informations de retour d'image en temps réel visualisées par un utilisateur, **caractérisé en ce que**
pour chacune parmi la ou les parties à corriger (106) sélectionnées, l'unité d'entrée utilisateur (101) envoie à l'unité de traitement (103) un signal de commande de correction destiné à effectuer une correction d'image sur la partie à corriger (106) dans un système de coordonnées où la grille/le groupe de grilles (107) est actuellement situé, le signal de commande de correction comprend un signal pour amener un ou plusieurs côtés de la partie à corriger (106) à fléchir, à s'allonger ou à s'incliner afin de corriger la partie à corriger (106) pour qu'elle devienne une ligne droite dans le système de coordonnées dans un champ visuel de l'utilisateur, et l'unité de traitement (103) effectue un traitement de calcul sur le signal de commande de correction pour obtenir des variations de la partie à corriger (106) avant et après correction dans le système de coordonnées,
l'unité de stockage (102) stocke la ou les combinaisons des variations obtenues pour la ou les parties à corriger, et
l'unité d'affichage (104) affiche une image après correction de la distorsion visuelle selon la ou les combinaisons des variations stockées dans l'unité de stockage (102).

2. Dispositif d'aide à la vision électronique monté sur tête selon la revendication 1 : l'unité de traitement (103) est configurée pour commander la grille/le groupe de grilles (107) du système de coordonnées pour se déplacer automatiquement d'une distance de pas déterminée dans une plage d'intervalle maximale de l'une quelconque des lignes de grille adjacentes.

3. Dispositif d'aide à la vision électronique monté sur tête selon la revendication 1 : l'unité d'entrée utilisateur (101) envoie à l'unité de traitement (103) un signal de commande de déplacement destiné à déplacer la grille/le groupe de grilles (107) dans le système de coordonnées, et l'unité de traitement (103) est configurée pour commander la grille/le groupe de grilles (107) affiché par l'unité d'affichage (104) pour se déplacer à la demande dans une plage d'intervalle maximale de l'une quelconque des lignes de grille adjacentes selon le signal de commande de déplacement.

4. Dispositif d'aide à la vision électronique monté sur tête selon la revendication 1, comprenant en outre : une unité de suivi oculaire (105), lorsque l'unité de suivi oculaire détecte un changement de la cible de regard de l'utilisateur, le système de coordonnées où se trouve la grille/le groupe de grilles (107) est déplacé de manière synchrone en suivant la cible de regard, et l'unité d'affichage (104) applique la ou les combinaisons des variations stockées dans l'unité de stockage (102) sur le système de coordonnées après le déplacement synchrone, et affiche l'image après correction de la distorsion visuelle.

5. Dispositif d'aide à la vision électronique monté sur tête selon la revendication 1 : l'unité d'entrée utilisateur (101) est une souris, une unité de commande à distance, un bouton, un dispositif de reconnaissance gestuelle ou un dispositif de reconnaissance vocale, et les variations sont une pluralité de tableaux de variables de coordonnées.

6. Dispositif d'aide à la vision électronique monté sur tête selon la revendication 1 : la distance entre des lignes de grille adjacentes de la grille/du groupe de grilles (107) est ajustée à la demande.

7. Procédé de correction de distorsion visuelle d'un dispositif d'aide visuelle électronique monté sur tête (100), le dispositif comprenant une unité d'entrée utilisateur (101), une unité de stockage (102), a une unité de traitement (103) et une unité d'affichage (104), le procédé comprenant les étapes suivantes :
A : l'unité d'affichage (104) affiche de manière mobile une grille/un groupe de grilles (107) ;
B : un utilisateur sélectionne une ou plusieurs parties à corriger (106) dans la grille/le groupe de grilles (107) par l'intermédiaire de l'unité d'entrée utilisateur (101) selon les informations de retour d'image en temps réel visualisées par l'utilisateur sur l'unité d'affichage (104) ; le procédé comprend :
C : pour chacune parmi la ou les parties à corriger (106), l'unité d'entrée utilisateur (101) envoie à l'unité de traitement (103) un signal de commande de correction destiné à effectuer une correction d'image sur la partie à corriger (106) dans un système de coordonnées où la grille/le groupe de grilles (107) est actuellement situé, le signal de commande de correction comprend un signal pour amener un ou plusieurs côtés de la partie à corriger (106) à fléchir, à s'allonger ou à s'incliner afin de corriger la partie à corriger (106) pour qu'elle devienne une ligne droite dans le système de coordonnées dans un champ visuel de l'utilisateur ;
D : pour chacune parmi la ou les parties à corriger (106), l'unité de traitement (103) effectue un traitement de calcul sur le signal de commande de correction pour obtenir des variations de la partie à corriger (106) avant et après correction dans le système de coordonnées ;
E : l'unité de stockage (102) stocke la ou les combinaisons des variations obtenues pour la ou les parties à corriger (106) ; et
F : l'unité d'affichage (104) affiche une image après correction de la distorsion visuelle selon la ou les combinaisons des variations stockées dans l'unité de stockage (102).

8. Procédé de correction de distorsion visuelle du dispositif d'aide à la vision électronique monté sur tête selon la revendication 7, comprenant en outre :
une étape C1 entre les étapes C et D : l'unité de traitement (103) commande la grille/le groupe de grilles (107) du système de coordonnées pour se déplacer automatiquement d'une distance de pas déterminée dans la plage d'intervalle maximale de l'une quelconque des lignes de grille adjacentes pour une correction automatique supplémentaire de toutes les parties à corriger (106) du système de coordonnées.

9. Procédé de correction de distorsion visuelle du dispositif d'aide à la vision électronique monté sur tête selon la revendication 7, comprenant en outre :
une étape C1 entre les étapes C et D : l'unité d'entrée utilisateur (101) envoie à l'unité de traitement (103) un signal de commande de déplacement destiné à déplacer la grille/le groupe de grilles (107) dans le système de coordonnées, et l'unité de traitement (103) commande la grille/le groupe de grilles (107) affiché par l'unité d'affichage (104) pour se déplacer à la demande dans une plage d'intervalle maximale de l'une quelconque des lignes de grille adjacentes selon le signal de commande de déplacement, de manière à corriger davantage toutes les parties à corriger (106) du système de coordonnées à la demande.

10. Procédé de correction de distorsion visuelle du dispositif d'aide à la vision électronique monté sur tête selon la revendication 7, **caractérisé en ce que** : le dispositif comprend en outre une unité de suivi oculaire (105), lorsque l'unité de suivi oculaire détecte un changement de la cible de regard de l'utilisateur, le système de coordonnées où se trouve la grille/le groupe de grilles (107) est déplacé de manière synchrone en suivant la cible de regard, et l'unité d'affichage (104) applique la ou les combinaisons des variations stockées dans l'unité de stockage (102) sur le système de coordonnées après le déplacement synchrone, et affiche l'image après correction de la distorsion visuelle.

11. Procédé de correction de distorsion visuelle du dispositif d'aide visuelle électronique monté sur tête selon la revendication 7, **caractérisé en ce que** : à l'étape B, l'unité d'entrée utilisateur (101) est une souris, une unité de commande à distance, un bouton, un dispositif de reconnaissance gestuelle ou un dispositif de reconnaissance vocale, et le bord extérieur de la partie à corriger (106) est sélectionné par l'unité d'entrée utilisateur (101) pour sélectionner la partie à corriger (106).

12. Procédé de correction de distorsion visuelle du dispositif d'aide à la vision électronique monté sur tête selon la revendication 8 ou 9, comprenant en outre :
une étape de confirmation permettant à l'utilisateur de confirmer le signal de commande de correction entre l'étape C et l'étape C1.

13. Procédé de correction de distorsion visuelle du dispositif d'aide à la vision électronique monté sur tête selon la revendication 7, **caractérisé en ce que** : la distance entre des lignes de grille adjacentes de la grille/du groupe de grilles (107) est ajustée à la demande.

14. Procédé de correction de distorsion visuelle du dispositif d'aide à la vision électronique monté sur la tête selon la revendication 7**,**
**caractérisé en ce que** : l'unité de stockage (102) à l'étape E stocke les combinaisons des variations de la ou des parties à corriger (106) de différents utilisateurs ou du même utilisateur à différents moments, et répète les étapes A à F en fonction de l'appel de la ou des combinaisons.
